# EUROPEAN PATENT APPLICATION

(11) **EP 4 321 087 A2**
(43) Date of publication of application: **14.02.2024**
(21) Application number: 23185435.7
(22) Date of filing: 13.07.2023
(51) Int. Cl.: A61B 5/00, A41D 1/00

(54) **TRANSMISSION DEVICE AND WEARABLE WITH TRANSMISSION DEVICE**

(30) Priority: 20.07.2022 TW 111127282
(71) Applicant: Chimera Multinational Co., Ltd, Taipei City (TW)
(72) Inventor: YAO, MING-HSIEN, Taipei City (TW); KAO, CHIH HSIUNG, Taipei City (TW)
(74) Representative: Lermer, Christoph

(57) **Abstract**

The invention relates to a transmission device applied to a smart wearable and a wearable with the transmission device. The transmission device has an outer layer, a thermal-bonding layer and at least one transmission line, the transmission line is capable of transmitting electric power and signals; the transmission device is attached to the wearable to provide electric power transmission or signal transmission required by the smart wearable. Two ends of the transmission device can be respectively connected with a sensing device and a battery device to realize physiological detection functions of the smart wearable. The transmission device is easy to manufacture and easy to be combined with the wearable.

## Description

### FIELD OF THE INVENTION

The invention relates to a transmission device for transmitting electric power and/or signals, and the transmission device is applied to textiles, fabrics or wearables. The invention also relates to a smart wearable with a transmission device.

### DESCRIPTION OF THE RELATED ART

Smart wearables, commonly known as smart clothes, can detect physiological information, such as body temperature, heart rate, blood pressure, etc., and transmit the physiological information to a smart device to monitor and keep track of physical conditions.

The smart clothes are equipped with conductive materials for transmitting signals. Existing conductive materials include nano-silver fibers, conductive silver plasma, etc. The more times the smart clothes made of such conductive materials are washed with water, the greater the electric resistance, causing problems in signal transmission.

Furthermore, due to the expensive material cost of conductive materials such as nano-silver fibers or conductive silver plasma, and the high manufacturing cost and high manufacturing technology threshold of the smart clothes made of such conductive materials, the prices of the smart clothes are expensive, which is not conducive to popularization. In addition, nano-silver fibers or conductive silver plasma cannot be modularly disposed on the smart clothes.

### SUMMARY OF THE INVENTION

One object of the invention is to provide a transmission device applied to a smart wearable, the transmission device is washable, and an electric resistance thereof does not increase due to increase in a number of times of washing with water.

One object of the invention is to provide a transmission device applied to a smart wearable, the transmission device is easy to manufacture and has low manufacturing cost.

One object of the invention is to provide a transmission device applied to a smart wearable, the transmission device can be modularized and easily combined with human wearables.

One object of the invention is to provide a wearable with the transmission device, the wearable is washable, has low manufacturing cost, is easy to manufacture, and is conducive to popularization and use.

One object of the invention is to provide a wearable with the transmission device, and the transmission device can be modularly manufactured.

One object of the invention is to provide a wearable with the transmission device, the transmission device can be easily combined with and separated from a sensing device and/or a battery device, the sensing device and the battery device can be modularly manufactured, and can be modularly connected with the transmission device.

The transmission device provided by the invention comprises:
an outer layer;
a thermal-bonding layer disposed on a surface of the outer layer, capable of producing viscosity when being heated; and
at least one transmission line disposed on the transmission device and capable of transmitting electric power or/and signals, the thermal-bonding layer is located between the outer layer and the transmission line or the transmission line is located between the outer layer and the thermal-bonding layer.

The transmission device of the invention is washable, and an electric resistance of the transmission line thereof does not change due to a number of times of washing. The transmission device is easy to manufacture, has low manufacturing cost, and can be modularly manufactured to have a specific length for installation on smart clothes (wearables) of different sizes or on different positions of the smart clothes.

Two ends of the transmission line are exposed at two ends of the transmission device in order to electrically connect with specific devices. Two ends of the transmission device can be provided with an interface respectively; two ends of the transmission line are respectively exposed at the two interfaces in order to facilitate connection with a sensing device, a battery device or other devices.

The transmission line can be fixed to the thermal-bonding layer through sewing threads, and the sewing threads can be made of dissolvable materials, so that after the transmission device is disposed on the smart clothes (wearables), the sewing threads can be dissolved in water or a solvent.

A material of the thermal-bonding layer can be selected from low-temperature thermoplastic polyurethane (TPU), and a material of the outer layer can be selected from high-temperature thermoplastic polyurethane (TPU), plastic film, rubber film, leather, fabric or textile, when the transmission device is attached to the smart clothes, a temperature applied to the thermal-bonding layer to make the thermal-bonding layer viscous is lower than a melting point of the outer layer.

The outer layer of the transmission device can be plastic film, rubber film, leather, fabric or textile. The transmission line can be: conductive fiber, conductive yarn, metal conducting wire, metal conducting wire with insulating layer, printed metal line or a transmission line formed by a base layer and a conductive layer..

In implementation, the transmission device comprises at least one flexible composite line; and the transmission line disposed in the flexible composite line; the thermal-bonding layer is located between the outer layer and the flexible composite line or the flexible composite line is located between the outer layer and the thermal-bonding layer.

In implementation, the transmission device comprises a soft film; and the transmission line is provided on the soft film; the thermal-bonding layer is located between the outer layer and the soft film or the soft film is located between the outer layer and thermal-bonding layer.

The wearable provided by the invention is a smart wearable provided for living organisms to wear, comprises: a wearing part, such as clothing body (main part of clothes), sleeve, leg of trousers or sock, worn on living organisms; further comprises:
a transmission device having an outer layer and at least one transmission line, the transmission line is located on one side of the outer layer and capable of transmitting electric power or/and signals;
the transmission device is attached to the wearing part; the transmission line is located between the outer layer and the wearing part.

The wearable of the invention is easy to manufacture and washable.

The wearing part of the wearable is further provided with a sensing device and a battery device, the sensing device is electrically connected to one end of the transmission line for sensing physiological information of living organisms; the battery device is electrically connected to another end of the transmission line to provide electrical energy to the sensing device via the transmission line. One or more than one of the transmission devices, one or more than one of the sensing devices and one or more than one of the battery devices can be disposed on the wearing part.

Two ends of the transmission line are exposed at two ends of the transmission device; the sensing device and the battery device are respectively connected to two ends of the transmission line.

In implementation, the sensing device has a base and a sensor, the base is disposed on the wearing part and electrically connected to one end of the transmission line; the sensor is detachably connected to the base and directly or indirectly electrically connected to one end of the transmission line; one or a plurality of sensing units and one or a plurality of wireless transmission units are provided in the sensing device, preferably disposed in the sensor; the sensing unit is used to sense physiological information of living organisms; the wireless transmission unit is used to transmit the physiological information to smart devices or cloud.

The battery device has a battery holder and a battery, the battery holder is disposed on the wearing part and connected to another end of the transmission line; the battery is detachably loaded into the battery holder, and electrical energy of the battery is transmitted to the sensing device via the transmission line.

In implementation, the transmission device of the wearable has a thermal-bonding layer combined with a surface of the outer layer; the thermal-bonding layer is located between the outer layer and the transmission line or the transmission line is located between the outer layer and the thermal-bonding layer.

The transmission device of the wearable has at least one flexible composite line; and the transmission line disposed in the flexible composite line. Alternatively, the transmission device of the wearable has at least one soft film; and the transmission line disposed on the soft film.

### BRIEF DESCRIPTION OF DRAWINGS

The invention can be understood from the following descriptions and drawings of several preferred embodiments, in which:
FIG. 1 is a perspective view of an implementation mode of a transmission device of a first preferred embodiment of the invention, wherein a transmission line faces upward and an outer layer faces downward.
FIG. 2 is a perspective view of another implementation mode of the transmission device of the first preferred embodiment of the invention, wherein the outer layer faces upward and the transmission line faces downward.
FIG. 3 is an exploded perspective view of FIG. 1.
FIG. 4 is a perspective view of the transmission device of FIG. 1 thermally adhered to a fabric.
FIG. 5 is a cross-sectional view showing that the transmission device has not yet adhered to the fabric.
FIG. 6 is a cross-sectional view of section line 6-6 in FIG. 4.
FIG. 7 is a perspective view of an implementation mode of the transmission device of a second preferred embodiment of the invention, wherein the transmission line faces upward and the outer layer faces downward.
FIG. 8 is a cross-sectional view of section line 8-8 in FIG. 7, and the outer layer is not shown in the figure.
FIG. 9 is a cross-sectional view of the transmission device of FIG. 7 thermally bonded to the fabric.
FIG. 10 shows that the transmission device of the invention is thermally bonded on the fabric, and two ends of the transmission device are respectively connected to a sensing device and a battery device.
FIG. 11 is a cross-sectional view of section line 11-11 in FIG. 10.
FIG. 12 is a cross-sectional view of section line 12-12 in FIG. 10.
FIG. 13 is a cross-sectional view of the transmission device, the sensing device and the battery device, and shows a state after removing a sensor and a battery.
FIG. 14 is a schematic diagram of a wearable with the transmission device of a preferred embodiment of the invention.
FIG. 15 is a perspective view of the transmission device of a third preferred embodiment of the invention, and the transmission device is bonded on the fabric.
FIG. 16 is an exploded perspective view of FIG. 15.
FIG. 17 is a cross-sectional view of section line 17-17 in FIG. 15.
FIG. 18 is the transmission device of a fourth preferred embodiment of the invention and the transmission device is bonded on the fabric.
FIG. 19 is an exploded perspective view of FIG. 18.
FIG. 20 is a perspective view of the transmission device of a fifth preferred embodiment of the invention, and the transmission device is bonded on the fabric.
FIG. 21 is an exploded perspective view of FIG. 20.
FIG. 22 is a perspective view of the transmission device of a sixth preferred embodiment of the invention, and the transmission device is bonded on the fabric and has an interface at one end.
FIG. 23 is a transverse cross-sectional view of the transmission device of a seventh preferred embodiment of the invention.
FIG. 24 is a transverse cross-sectional view of the transmission device of an eighth preferred embodiment of the invention.
FIG. 25 is a transverse cross-sectional view of the transmission device of a ninth preferred embodiment of the invention.
FIG. 26 is a transverse cross-sectional view of the transmission device of a tenth preferred embodiment of the invention.

### DETAILED DESCRIPTION OF THE INVENTION

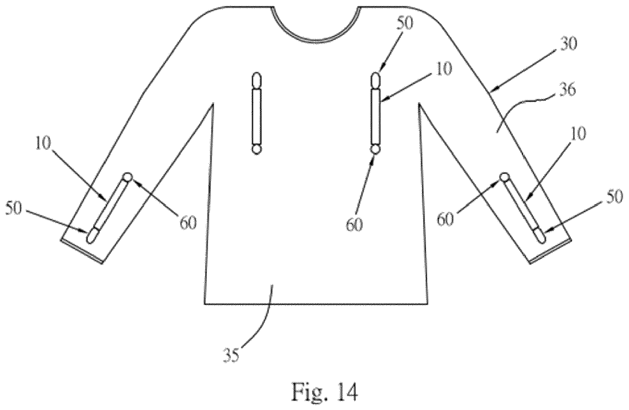

The invention provides a transmission device 10, which can be applied to various kinds of fabrics (including woven and non-woven), textiles and clothes worn by the human body, such as clothes, trousers, hats, caps, socks, gloves and the like. Wearables described in this specification comprise fabrics, textiles, clothes, trousers, hats, caps, socks, gloves and the like made of fabrics or textiles for the human body to wear. The invention further provides a smart wearable, the transmission device 10 of the invention is disposed on clothes worn by the human body to form the smart wearable, such as a smart clothing 30 shown in FIG. 14. The smart clothing hereinafter represents the smart wearable described in the invention, and reference number 10 represents the transmission device of each embodiment.

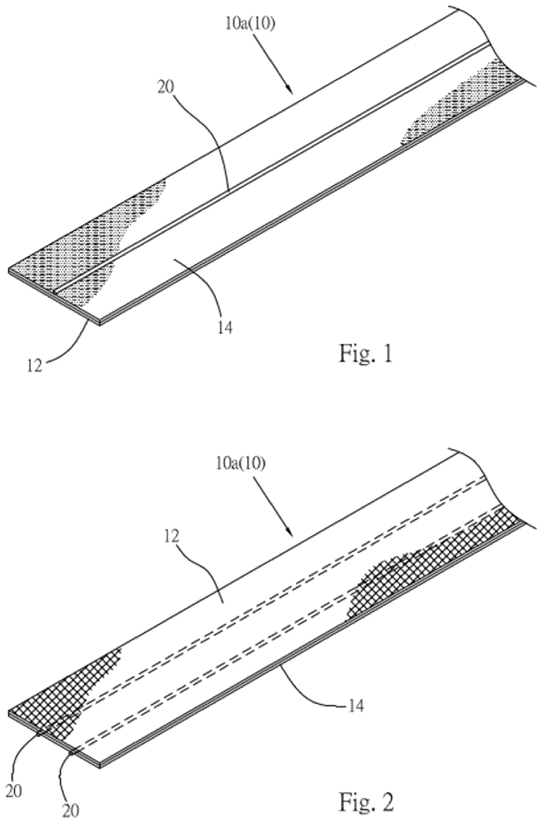

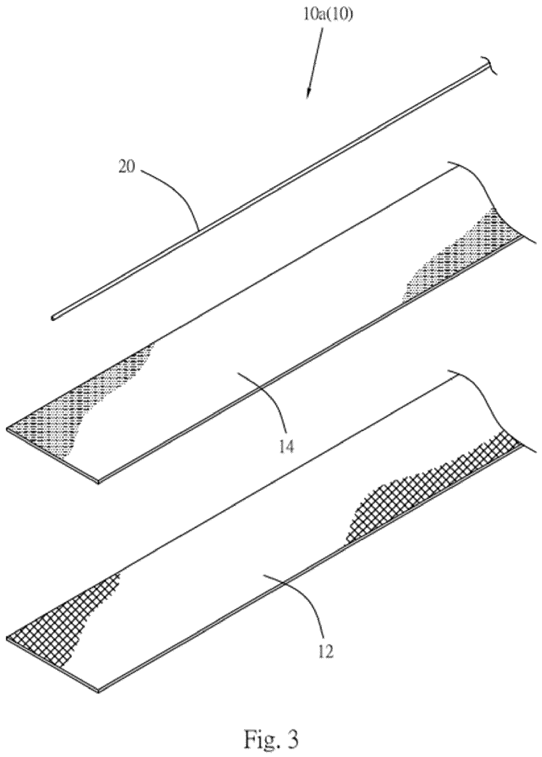

FIGS. 1 to 3 show a transmission device 10a (10) according to a first preferred embodiment of the invention, which comprises: a thin outer layer 12, an adhesive layer and one transmission line 20 or a plurality of transmission lines 20. The transmission device 10 is a sheet-like object, which can be made into a long strip and has a considerable length. After being made, the transmission device 10 can be rolled up into a roll, but the transmission device 10 is not limited to being a long strip.

The adhesive layer is a thermal-bonding layer 14, in this embodiment, a low-temperature hot-melt adhesive is selected, which is a thermoplastic polyurethane (TPU) material, and becomes adhesive when being heated. The thermal-bonding layer 14 is coated or bonded on a surface of the outer layer 12 to form an inner layer of the transmission device 10a.

The transmission line 20 is disposed on a surface of the thermal-bonding layer 14, and one transmission line 20 (as shown in FIG. 1) or transmission lines 20 (as shown in FIG. 2) can be disposed on the surface of the thermal-bonding layer 14 as required. The transmission line 20 is located on an inner side (inner surface) of the outer layer 12, and the thermal-bonding layer 14 is located between the outer layer 12 and the transmission line 20. The transmission line 20 is capable of transmitting signals and/or electric power, the transmission line 20 is a conductive wire, such as conductive fiber, conductive yarn (also called conductive thread, conductive line), or various hard and soft metal conductive wires, such as gold wire, copper wire, and the conductive wire has a small diameter, for example, 0.05~0.3mm; the transmission line 20 can also be a metal conductive wire with an insulating layer, such as enameled copper wire with a diameter of 0.1~0.5mm. The transmission line 20 is not limited to the aforesaid, any wire capable of transmitting signals and electricity can be used as the transmission line 20 of the invention.

The outer layer 12 can be various films or thin objects, such as high-temperature TPU, various plastic films or rubber films, natural or artificial leather, fabrics, etc. TPU is a polymer material with plastic and rubber properties. In this embodiment, the outer layer 12 is made of high-temperature TPU with a melting point higher than 160°C, preferably 180-210°C, which is higher than a melting point of the thermal-bonding layer 14 (low-temperature TPU): 80-150°C. The outer layer 12 has toughness and does not fracture easily. If the outer layer 12 is made of high-temperature TPU, molecular chain and molecular weight thereof are higher than those of low-temperature TPU, which has excellent toughness and does not fracture easily. Plastic film and leather also have excellent toughness.

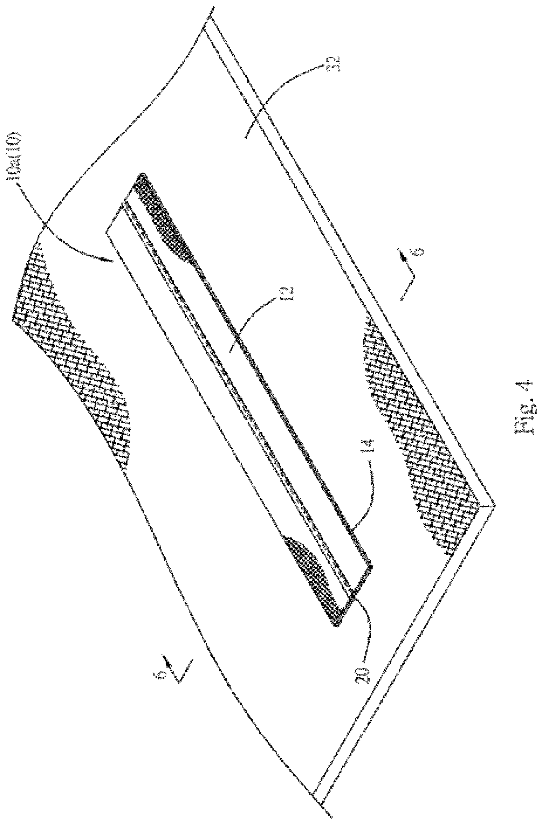

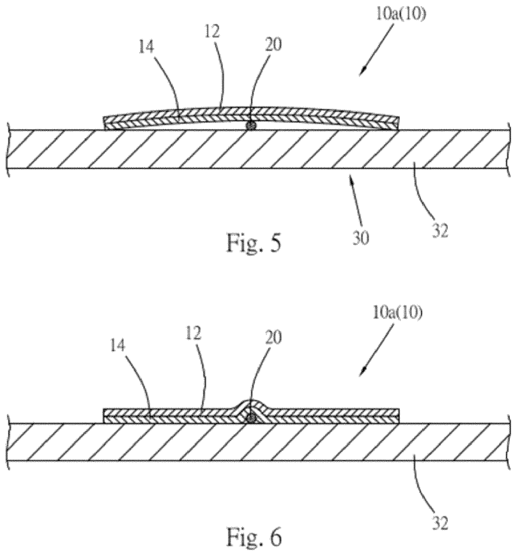

The smart clothing 30 has a wearing part worn on the human body, such as a clothing body 35 of clothing, a sleeve 36 of clothing shown in FIG. 14, or legs of trousers, etc. The transmission device 10 with an appropriate length, such as but not limited to 5 to 10 cm, is thermal-bonded on a fabric 32 of the wearing part of the smart clothing 30, as shown in FIG. 4 and FIG. 6, and disposed on an inner surface of the smart clothing 30. The transmission device 10 cannot be seen from the appearance. Please refer to FIG. 5, in thermal-bonding, the fabric 32 is contacted with the thermal-bonding layer 14, and then heat is applied to the transmission device 10 with a hot-pressing device to adhere the transmission device 10 on the fabric 32. If the outer layer 12 is made of high-temperature hot-melt adhesive, a temperature of hot pressing is lower than a melting point of high-temperature hot-melt adhesive, for example, 130-140°C, so that the thermal-bonding layer 14 made of low-temperature TPU is heated and melted to produce viscosity. The outer layer 12 made of high-temperature TPU will not melt, and will remain in a solid state. The transmission device 10 is adhered on the fabric 32 by a viscosity of the thermal-bonding layer 14. As shown in FIG. 6, the transmission line 20 is fixed on the fabric 32, hidden between the fabric 32 and the thermal-bonding layer 14 and the outer layer 12, and is not exposed. The transmission device 10, including the transmission line 20, is flexible and capable of flexing along with the fabric 32 without affecting movement of a wearer.

After the transmission device 10 is disposed on the fabric 32 of the smart clothing 30, the transmission line 20 can become a transmission medium of the smart clothing 30 for transmitting signals or/and electric power.

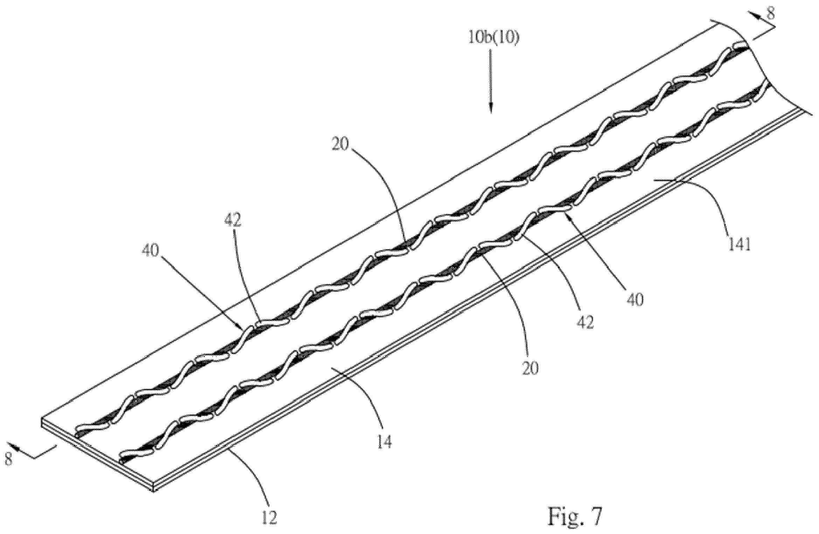

FIG. 7 shows a transmission device 10b (10) of a second preferred embodiment of the invention, which also comprises an outer layer 12, a thermal-bonding layer 14 and one or a plurality of transmission lines 20, two transmission lines 20 are shown in the figure.

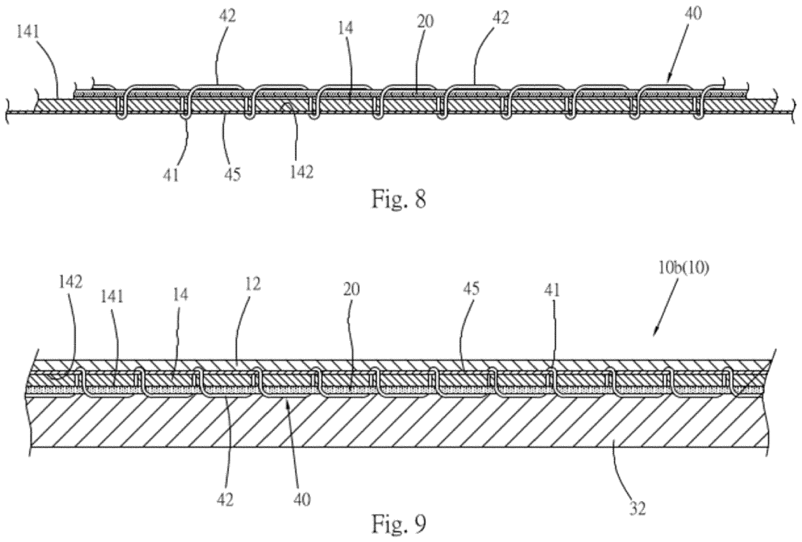

In this embodiment, each of the transmission lines 20 is fixed on the thermal-bonding layer 14 by means of sewing with at least one sewing thread. Please refer to FIGS. 7 and 8, each of the transmission lines 20 is fixed on a surface 141 of the thermal-bonding layer 14 by seam sections 42 of at least one first sewing thread 40; the first sewing thread 40 has a plurality of hooking portions 41 penetrating another surface 142 of the thermal-bonding layer 14 and hooked with at least one second sewing thread 45. A manner in which the transmission line 20 is fixed by the sewing thread is not limited to that shown in this embodiment. The outer layer 12 is disposed on the other surface 142 of the thermal-bonding layer 14.

Please refer to FIG. 9, the transmission device 10b is also adhered to an inner surface of a fabric 32 by thermal bonding, and the transmission line 20 is fixed on the fabric 32. The sewing threads 40, 45 can be made of polyvinyl alcohol (PVA), which is watersoluble. After the transmission device 10b is fixed on the smart clothing 30, the smart clothing 30 can be soaked in water to dissolve the sewing threads 40, 45 in the water, and only the transmission line 20, the heat bonding layer 14 and the outer layer 12 remain in the transmission device 10b, the transmission line 20 is capable of transmitting physiological data sensed by the smart clothing 30 and/or electric power required by the smart clothing 30.

The transmission device 10 of the invention is capable of transmitting electric power and signals. The transmission device 10 is not only easy to manufacture and can be modularized with various specific lengths, a structure thereof is also conducive to connection with a sensing device and a battery device of the smart clothing 30, so that the sensing device and the battery device can be modularly manufactured, and can connect with the transmission device 10.

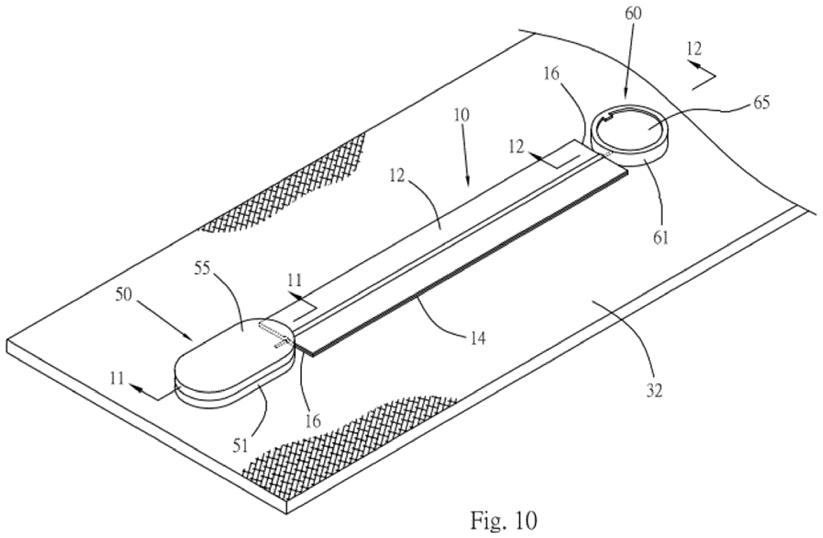

Please refer to FIG. 10 to FIG. 12, taking the transmission device 10 of the first preferred embodiment as an illustration, two ends of the transmission device 10 can be respectively connected to a sensing device 50 and a battery device 60, and the transmission device 10, the sensing device 50 and the battery device 60 are disposed on the inner surface of the fabric 32 of the smart clothing 30 together.

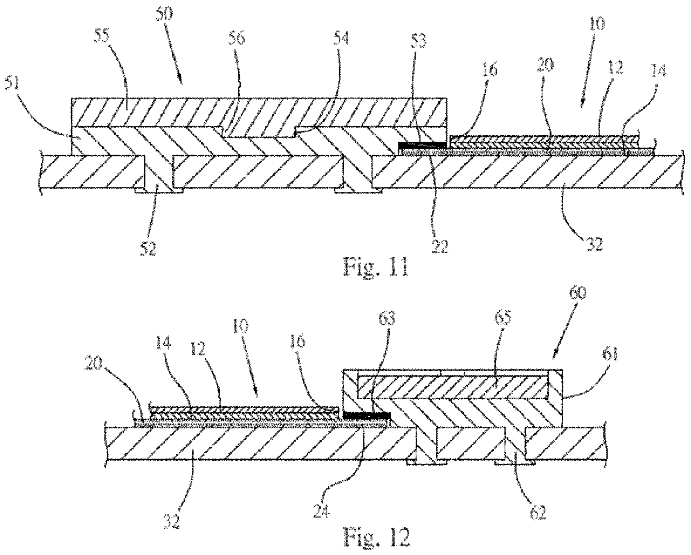

Please refer to FIG. 11 and FIG. 12, the two ends of the transmission device 10 can be respectively provided with an interface 16 conveniently, that is, appropriate lengths of the outer layer 12 and the thermal-bonding layer 14 are cut off to form the interfaces 16, and two ends of the transmission line 20 are exposed at the two interfaces 16 respectively.

The sensing device 50 has a base 51 and a sensor 55, the base 51 is disposed on the fabric 32 and fixed on the fabric 32 by one foot 52 or a plurality of feet 52. A contact 53 is disposed on a peripheral surface of the base 51 and electrically connected to a first end 22 of the transmission line 20. The sensor 55 is connected to the base 51 in a detachable manner, for example, the sensor 55 is magnetically attracted on the base 51 through a magnetic element (not shown), or the sensor 55 is detachably fastened with the base 51 using a fastener. The sensing device 50 is provided with one or a plurality of sensing units (not shown), one or a plurality of wireless transmission units (not shown) and a signal processing unit for processing signals. Preferably, the sensing unit, the wireless transmission unit and the signal processing unit are installed in the sensor 55. The sensing unit comprises, but is not limited to, electrode plate, infrared sensing unit, etc., which is capable of detecting various physiological data and bioelectrical signals of the human body, including: body temperature, heart rate, electrocardiogram, blood pressure, blood oxygen, brain pressure, pH value of sweat, etc. The wireless transmission unit comprises, but is not limited to, WIFI module, Bluetooth module, LoRa wireless module, RF wireless module, NFC module, etc., which is capable of wirelessly transmitting physiological data measured by the sensor 55 to smart devices, cloud, etc., via APP (application program). When the sensor 55 is connected to the base 51, an electrical contact 56 of the sensor 55 contacts an electrical contact 54 of the base 51, and the contact 53 of the base 51 is electrically connected to the electrical contact 54; electric power of the battery device 60 is transmitted from the transmission line 20 to the base 51, and then transmitted from the base 51 to the sensor 55 to provide electric power required for the sensor 55 to operate. In implementation, when the sensor 55 is combined with the base 51, the electrical contact 56 of the sensor 55 can be made to directly contact the first end 22 of the transmission line 20 so that the sensor 55 is directly electrically connected to the transmission line 20.

The battery device 60 has a battery holder 61 and a battery 65. The battery holder 61 is fixed on the fabric 32 by one foot 62 or a plurality of feet 62. The battery holder 61 is provided with conductive plates (not shown) for connecting with positive and negative poles of the battery 65. A contact 63 is disposed on a peripheral surface of the battery holder 61 and electrically connected to a second end 24 of the transmission line 20. The battery 65 is preferably a button battery, and disposed in the battery holder 61. Electrical energy of the battery 65 is transmitted to the base 51 via the transmission line 20 of the transmission device 10 and supplied to the sensor 55.

The smart clothing 30 in FIG. 14 shows that the transmission devices 10 are installed, and two ends of each of the transmission devices 10 are respectively connected to the sensing device 50 and the battery device 60. When a user wears the smart clothing 30, the sensing device 50 can detect various physiological data of the human body, and wirelessly transmit the detected physiological data to smart devices or cloud to monitor a wearer's physiological conditions, which is suitable for general health detection and medical purposes.

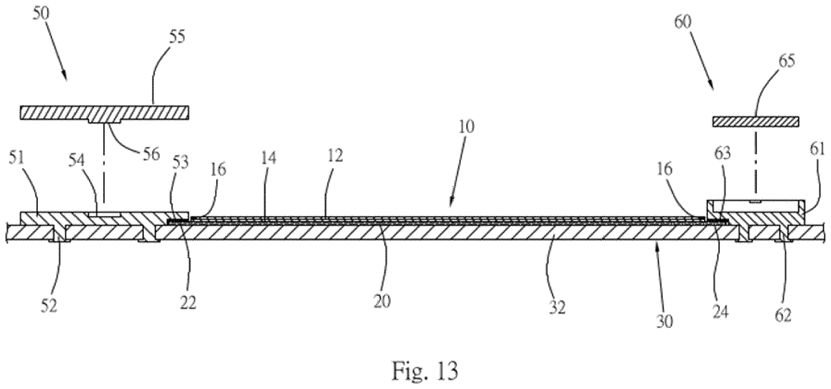

When the smart clothing 30 needs to be washed, as shown in FIG. 13, the sensor 55 and the battery 65 are removed from the base 51 and the battery holder 61 respectively, and the sensor 55 and the battery 65 are put back into the base 51 and the battery holder 61 respectively when the smart clothing 30 needs to be worn to restore sensing functions of the smart clothing 30.

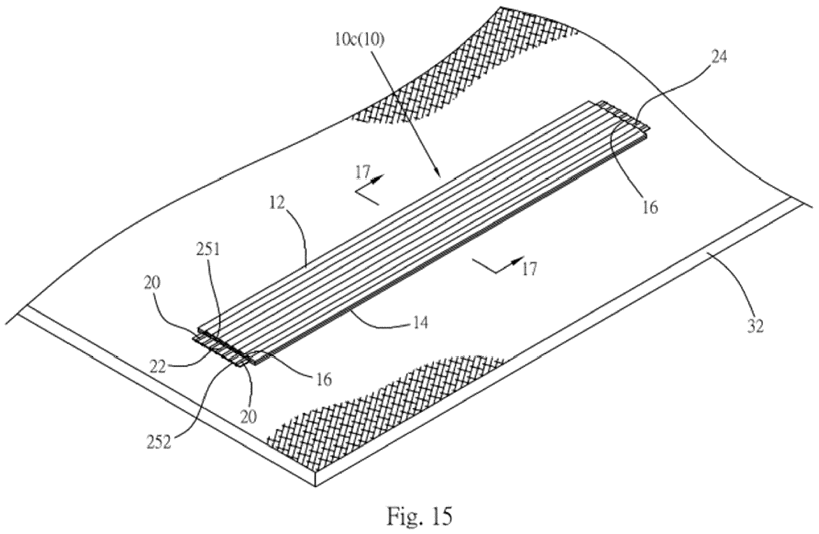

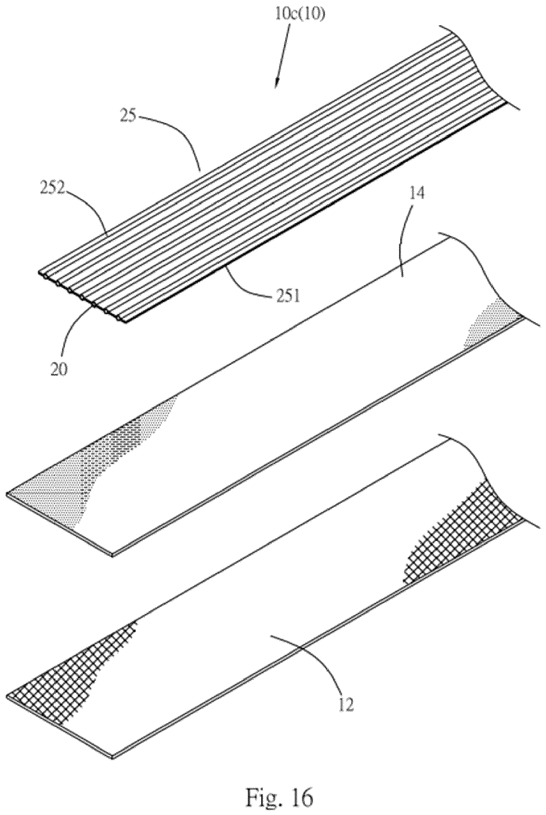

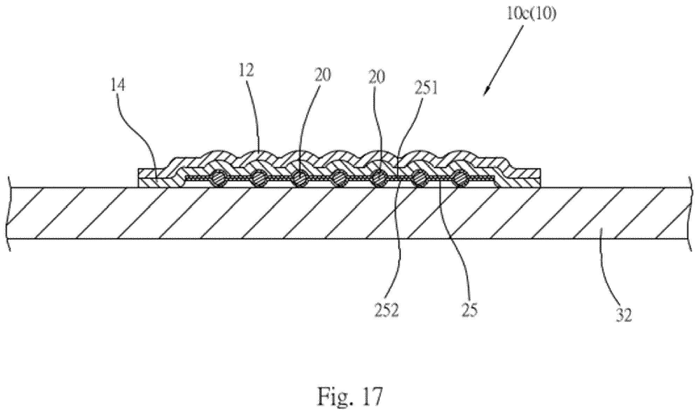

FIGS. 15 to 17 show a transmission device 10c (10) of a third preferred embodiment of the invention, which also comprises: an outer layer 12, a thermal-bonding layer 14 and at least one transmission line 20. Wherein, the transmission device 10c further comprises at least one flexible composite line 25, the flexible composite line 25 has two soft film layers 251, 252 combined with each other; and one or a plurality of transmission lines 20 fixed in the two film layers 251, 252. The flexible composite line 25 is soft, thin, and can be bent at will, and is disposed on a surface of the thermal-bonding layer 14. The transmission device 10c is attached on a fabric 32 in a thermal-bonding manner, and can be flexed along with the fabric 32. The transmission device 10c is adhered on the fabric 32 by viscosity of the thermal-bonding layer 14, and the transmission line 20 is located between the outer layer 12 and the fabric 32. The two film layers 251, 252 are soft, flexible and elastic film materials or polymers, such as high-temperature TPU, low-temperature TPU, medical grade polyvinyl chloride (PVC), etc., and the two film layers 251, 252 can be made of a same material or different materials. The flexible composite line 25 is soft and easy to bend, and will not cause discomfort or burden on wearing. If the film layers 251, 252 are made of high-temperature TPU or PVC, melting points thereof are higher than a melting point of the thermal-bonding layer 14. When the thermal-bonding layer 14 is heated and melted to generate viscosity, the film layers 251, 252 still remain solid and do not melt. If low-temperature TPU is used for the film layers 251, 252, the film layers 251, 252 can be melted together with the thermal-bonding layer 14.

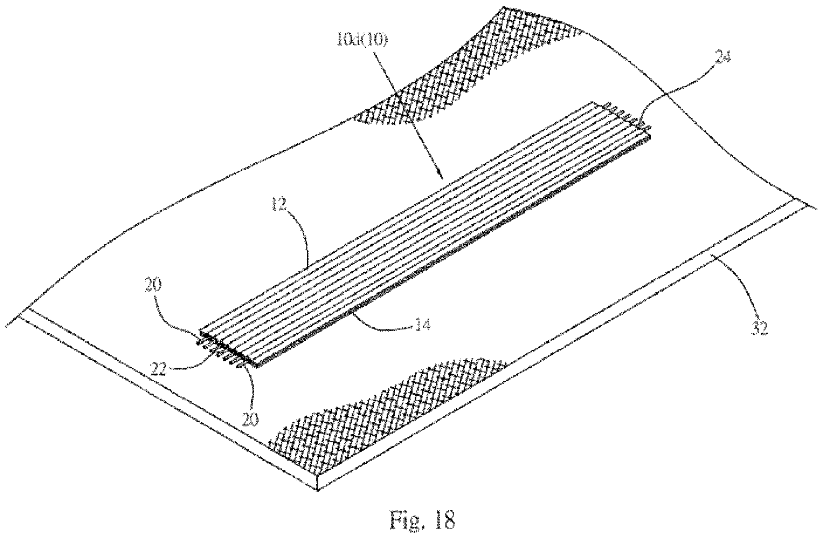

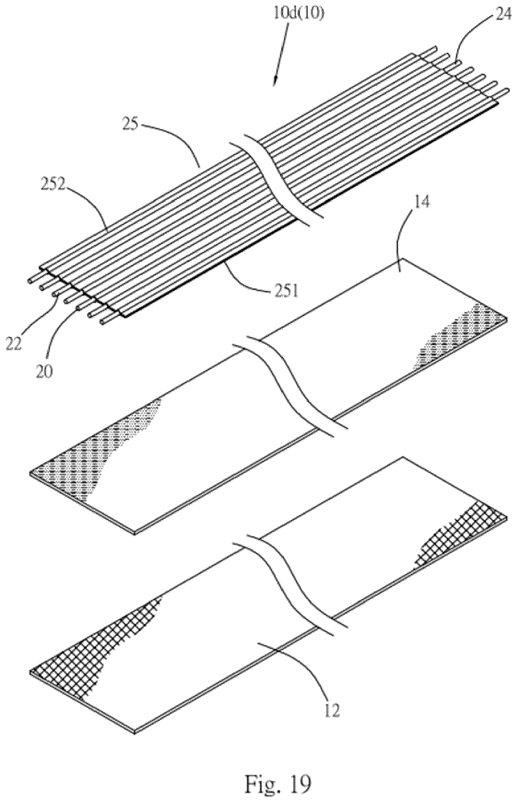

FIG. 18 and FIG. 19 show a transmission device 10d (10) of a fourth preferred embodiment of the invention, which also comprises an outer layer 12, a thermal-bonding layer 14, and at least one flexible composite line 25, one or a plurality of transmission lines 20 are disposed in two film layers 251, 252 of the flexible composite line 25. The flexible composite line 25 is disposed on a surface of the thermal-bonding layer 14. The transmission device 10d is thermally bonded on a fabric 32.

Please refer to FIG. 15, after the transmission device 10c (10) is disposed on a fabric 32, two interfaces 16 are respectively disposed at two ends of the transmission device 10c, so that the first end 22 and the second end 24 of the transmission line 20 are exposed at two ends of the transmission device 10c in order to electrically connect the sensing device and the battery device with two ends 22, 24 of the transmission line 20. A connection relationship between the sensing device and the battery device with the two ends 22, 24 of the transmission line 20 can be understood from FIG. 11 and FIG. 12.

Please refer to FIG. 18 for another implementation option in which when the flexible composite line 25 is manufactured, two ends 22, 24 of the transmission line 20 are exposed from the film layers 251, 252 in advance, and then the flexible composite line 25 is disposed on a surface of the thermal-bonding layer 14 to expose the two ends 22, 24 of the transmission line 20 at two ends of the transmission device 10d. Thereby, when the transmission device 10d is attached to a fabric 32, the two ends 22, 24 of the transmission line 20 are exposed in order to electrically connect with other devices without requiring to form the interface 16 by cutting.

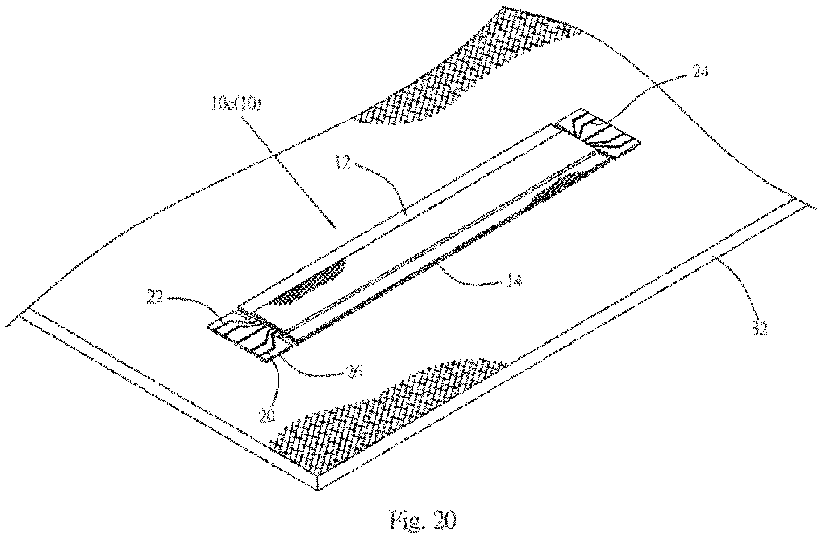

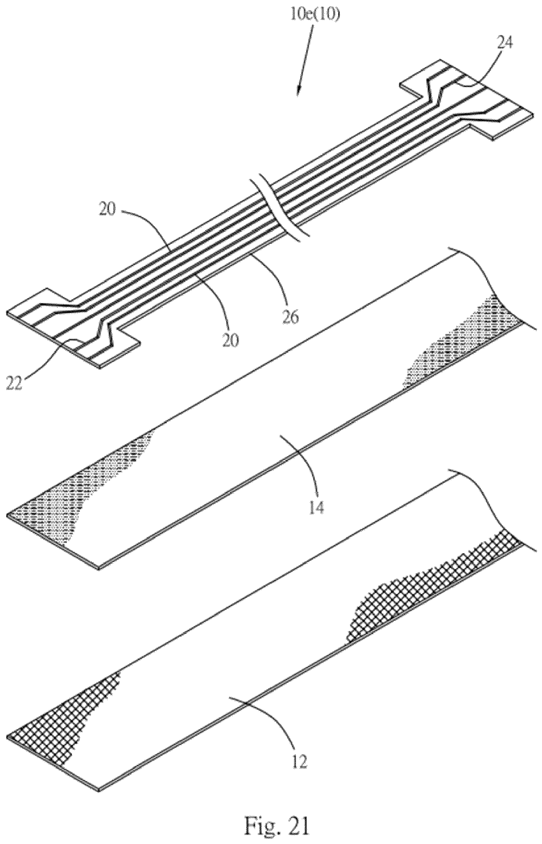

FIGS. 20 to 21 show a fifth preferred embodiment of a transmission device 10e (10) of the invention, comprising an outer layer 12, a thermal-bonding layer 14, and at least one soft film 26, the soft film 26 is made of high molecular polymers, such as TPU, medical grade PVC, which is thin, flexible and bendable. One or a plurality of printed metal lines are printed on the soft film 26 to form one or a plurality of transmission lines 20 of the invention, and two ends 22, 24 of the transmission line 20 are printed on two ends of the soft film 26. The soft film 26 is disposed on a surface of the thermal-bonding layer 14, and a length thereof is longer than that of the outer layer 12 and the thermal-bonding layer 14, so that two ends of the soft film 26 are directly exposed at two ends of the transmission device 10e. After the transmission device 10e is thermally bonded to a fabric 32, two ends 22, 24 of the transmission line 20 are exposed for electrically connecting with other devices. The transmission line 20 is located between the outer layer 12 and the fabric 32. A melting point of the soft film 26 is higher than a melting point of the thermal-bonding layer 14. When the thermal-bonding layer 14 melts and becomes viscous, the soft film 26 remains solid.

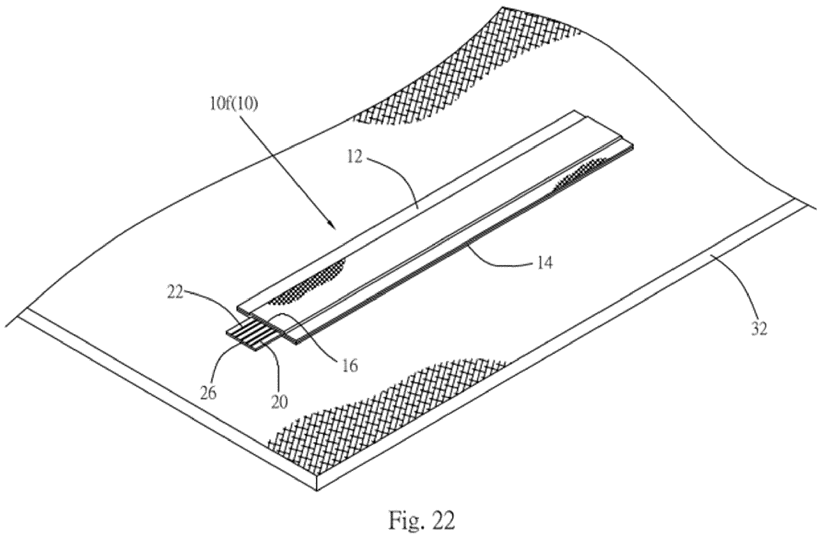

FIG. 22 shows a transmission device 10f (10), two ends of the soft film 26 do not expose the outer layer 12 and the thermal-bonding layer 14. After the transmission device 10f is attached to a fabric 32, two ends of the transmission device 10f are provided with the interfaces 16 respectively to expose two ends of the soft film 26 and two ends 22, 24 of the transmission lines 20 formed by the printed metal lines. In the figure, an interface 16 is provided at one of the ends of the transmission device 10f as an example, and the first ends 22 of the transmission lines 20 are exposed.

In the above-mentioned embodiments, the thermal-bonding layer 14 is located between the outer layer 12 and the transmission lines 20.

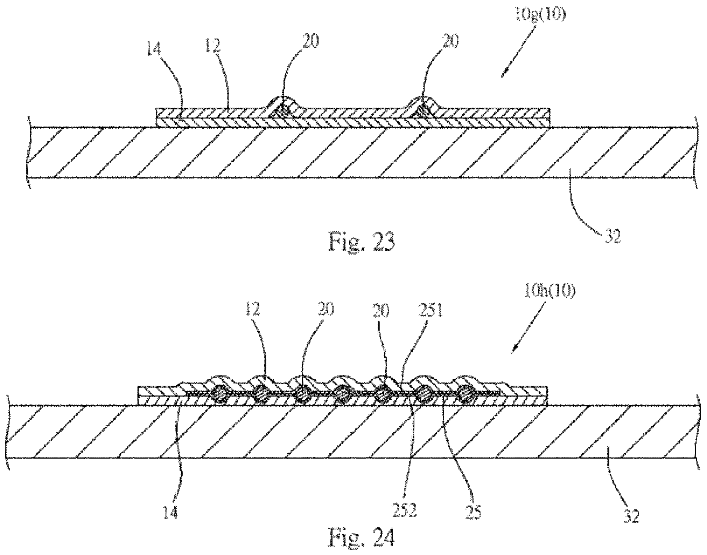

FIG. 23 shows a seventh preferred embodiment of a transmission device 10g (10) of the invention, the transmission device 10g can be the transmission device shown in FIG. 1, FIG. 2 or FIG. 7, a difference lies in: the transmission lines 20 are disposed between the outer layer 12 and the thermal-bonding layer 14. The transmission device 10g is attached on a fabric 32 through the thermal-bonding layer (adhesive layer) 14. Two ends of the transmission device 10g can be provided with the interfaces 16 to expose the two ends 22, 24 of the transmission lines 20 to transmit electrical energy or signals.

FIG. 24 shows an eighth preferred embodiment of a transmission device 10h (10) of the invention, the transmission device 10h is similar to the transmission device shown in FIG. 15 or FIG. 18, and has at least one flexible composite line 25, a difference lies in: the flexible composite line 25 is disposed between the outer layer 12 and the thermal-bonding layer 14. The transmission device 10h is attached on a fabric 32 through the thermal-bonding layer (adhesive layer) 14. The transmission device 10h can be as shown in the transmission device in FIG. 15, two interfaces 16 are respectively provided at two ends of the transmission device 10h to expose the two ends 22, 24 of the transmission lines 20; or as shown in the transmission device in FIG. 18, two ends of the transmission lines 20 are exposed by default at two ends of the transmission device 10h.

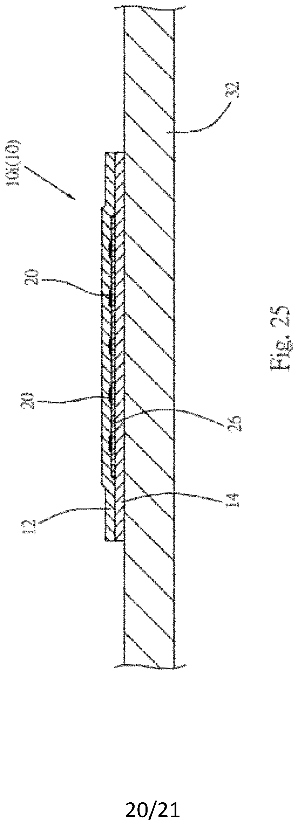

FIG. 25 shows a ninth preferred embodiment of a transmission device 101 (10) of the invention, the transmission device 10i is similar to the transmission device shown in FIG. 20 or FIG. 22, has at least one soft film 26, and the soft film 26 is printed with at least one printed metal line to form at least one transmission line 20, a difference lies in: the soft film 26 is disposed between the outer layer 12 and the thermal-bonding layer 14. The transmission device 10i is attached on a fabric 32 through the thermal-bonding layer (adhesive layer) 14. The transmission device 10i can be as shown in the transmission device in FIG. 20, two ends of the soft film 26 and two ends 22, 24 of the transmission lines 20 are exposed by default at two ends of the transmission device 101; or the transmission device 10i can be as shown in the transmission device in FIG. 22, two interfaces 16 are respectively provided at two ends of the transmission device 10i to expose two ends 22, 24 of the transmission lines 20.

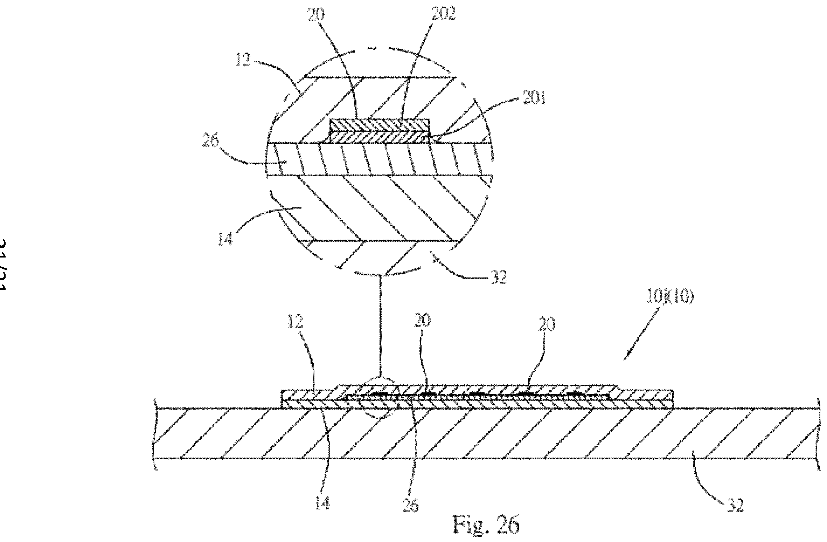

FIG. 26 shows a tenth preferred embodiment of a transmission device 10j (10) of the invention, the transmission device 10j is similar to the transmission device shown in FIG. 20 or FIG. 22, has at least one soft film 26, and one or a plurality of transmission lines 20 disposed on the soft film 26, wherein: each of the transmission lines 20 has a base layer 201 made of TPU material and a conductive layer 202 coated on the base layer 201. In this embodiment, the conductive layer 202 comprises conductive carbon black and graphene. The TPU soft film 26 is soft and elastic, so that the transmission lines 20 are capable of stretching along with the fabric 32.

The transmission device 10 (10a~10j) of the invention has many advantages. The transmission device 10 is washable, and an electric resistance of the transmission line 20 will not increase even after repeated washing. The smart clothing equipped with the transmission device is also washable, and sensing functions thereof will not be reduced or impaired due to repeated washing.

The transmission device 10 is easy to manufacture, and can be modularly manufactured with different lengths to fit different sizes of the wearable, or installed at different positions of the wearable. The transmission line 20, the outer layer 12, the thermal-bonding layer 14, the flexible composite line 25 and the soft film 26 can be modularly manufactured. The transmission device has low material cost and low manufacturing cost, and is easy to combine with the wearable, which can reduce a manufacturing cost of the smart clothing 30 to facilitate promotion and popularization of the smart clothing 30.

The two ends of the transmission device 10 can be easily made into the interfaces 16 for easy connection with the sensing device and the battery device, and the sensing device and the battery device can be modularly manufactured to be modularly connected with the interfaces of the transmission device. When the transmission line is provided by the flexible composite line and the soft film 26, two ends of the transmission line can be easily fabricated at two ends of the transmission device 10.

The transmission device of the invention can be connected with other types of devices to transmit electronic signals.

The invention makes it easy to separate the sensing device and the battery device from the transmission device and combine the sensing device and the battery device with the transmission device, thereby improving a practicability of the smart clothing.

The technology provided by the invention comprises fixing the transmission line on the transmission device by sewing, and the sewing thread can be made of dissolvable material, which can be dissolved in water or a solvent. After the transmission device is thermal-bonded to the smart clothing 30, the sewing thread can be dissolved to improve comfort of wearing.

Although the invention has been disclosed as above with the embodiments, it is not intended to limit the invention. A person having ordinary skill in the art to which the invention pertains can make various changes and modifications without departing from the spirit and scope of the invention. Therefore, scope of protection of the invention shall be subject to what is defined in the pending claims.

## Claims

1. A transmission device comprising:
an outer layer being plastic film, rubber film, leather, fabric or textile;
a thermal-bonding layer capable of producing viscosity when being heated, the thermal-bonding layer being combined on a surface of the outer layer; and
at least one transmission line disposed on the transmission device and capable of transmitting electric power or/and signals, the thermal-bonding layer being located between the outer layer and the transmission line or the transmission line being located between the outer layer and the thermal-bonding layer.

2. The transmission device as claimed in claim 1, wherein two ends of the transmission device is provided with an interface respectively; two ends of the transmission line are respectively exposed at the two interfaces.

3. The transmission device as claimed in claim 1, further comprising at least one sewing thread sewn on the thermal-bonding layer; the transmission line being fixed on the thermal-bonding layer by the sewing thread.

4. The transmission device as claimed in claim 3, wherein the sewing thread is dissolvable and capable of dissolving in water or a solvent.

5. The transmission device as claimed in any one of claims 1 to 4, wherein the thermal-bonding layer is low-temperature thermoplastic polyurethane (TPU), the outer layer is high-temperature thermoplastic polyurethane (TPU), plastic film, rubber film, leather, fabric or textile, a temperature applied to the thermal-bonding layer to make the thermal-bonding layer viscous is lower than a melting point of the outer layer.

6. The transmission device as claimed in any one of claims 1 to 5, wherein the transmission line is conductive fiber, conductive yarn, metal conducting wire, metal conducting wire with insulating layer, printed metal line or a transmission line formed by a base layer and a conductive layer.

7. The transmission device as claimed in claim 1 or 2, further comprising at least one flexible composite line; one or a plurality of the transmission lines disposed in the flexible composite line; the thermal-bonding layer being located between the outer layer and the flexible composite line or the flexible composite line being located between the outer layer and the thermal-bonding layer.

8. The transmission device as claimed in claim 7, wherein the flexible composite line comprises two soft film layers combined with each other; the transmission line is disposed between the two film layers.

9. The transmission device as claimed in claim 1 or 2, further comprising a soft film; the transmission line being disposed on the soft film; the thermal-bonding layer being located between the outer layer and the soft film or the soft film being located between the outer layer and the thermal-bonding layer.

10. The transmission device as claimed in claim 9, wherein the soft film is elastic; the at least one transmission line has a base layer and a conductive layer, the base layer is elastic; the conductive layer is disposed on the base layer.

11. A wearable provided for living organisms to wear, the wearable comprising a wearing part worn on living organisms; further comprising:
a transmission device having an outer layer and at least one transmission line, the transmission line being located on one side of the outer layer and capable of transmitting electric power or/and signals;
the transmission device being attached to a surface of the wearing part; the transmission line being located between the outer layer and the surface of the wearing part;
a sensing device and a battery device disposed on the wearing part, the sensing device being electrically connected to one end of the transmission line for sensing physiological information of living organisms; the battery device being electrically connected to another end of the transmission line to provide electrical energy to the sensing device via the transmission line;
the sensing device having a base and a sensor, the base being disposed on the wearing part and electrically connected to one of the ends of the transmission line; the sensor being detachably connected to the base and directly or indirectly electrically connected to one of the ends of the transmission line; one or a plurality of sensing units and one or a plurality of wireless transmission units being provided in the sensing device; the sensing unit being disposed in the sensor and being used to sense physiological information of living organisms; the wireless transmission unit being used to transmit the physiological information to smart devices or cloud; and
the battery device having a battery holder and a battery, the battery holder being disposed on the wearing part and connected to the other end of the transmission line; the battery being detachably loaded into the battery holder, and electrical energy of the battery being transmitted to the sensing device via the transmission line.

12. The wearable as claimed in claim 11, wherein two ends of the transmission device is provided with an interface respectively; the sensing device and the battery device are respectively connected to two ends of the transmission line through the two interfaces.

13. The wearable as claimed in claim 11 or 12, wherein a contact is disposed on a peripheral surface of the base and electrically connected to one end of the transmission line; the base and the sensor are respectively provided with an electrical contact, when the sensor is combined with the base, the electrical contact of the sensor contacts the electrical contact of the base; the electrical contact of the base is electrically connected to the contact.

14. The wearable as claimed in any one of claims 11 to 13, wherein the transmission device has a thermal-bonding layer combined to a surface of the outer layer; the thermal-bonding layer is located between the outer layer and the transmission line or the transmission line is located between the outer layer and the thermal-bonding layer; the transmission device is adhered to the wearing part by the thermal-bonding layer.

15. The wearable as claimed in claim 14, wherein the thermal-bonding layer is low-temperature thermoplastic polyurethane (TPU), and the outer layer is high-temperature thermoplastic polyurethane (TPU), plastic film, rubber film, leather, fabric or textile, a temperature applied to the thermal-bonding layer to make the thermal-bonding layer viscous is lower than a melting point of the outer layer.
